# EUROPÄISCHE PATENTSCHRIFT

(11) **EP 0 971 681 B1**
(45) Veröffentlichungstag und Bekanntmachung des Hinweises auf die Patenterteilung: **02.05.2003**
(21) Anmeldenummer: 98908111.2
(22) Anmeldetag: 25.02.1998
(51) Int. Cl.: A61K 7/13

(54) **MITTEL UND VERFAHREN ZUM FÄRBEN UND TÖNEN KERATINISCHER FASERN**
MEANS AND METHOD FOR DYING AND COLORING KERATIN FIBERS
PRODUIT ET PROCEDE POUR TEINTER ET COLORER DES FIBRES DE KERATINE

(30) Priorität: 03.03.1997 DE 19708497
(43) Veröffentlichungstag der Anmeldung: 19.01.2000
(73) Patentinhaber: Hans Schwarzkopf GmbH & Co. KG, 22763 Hamburg (DE)
(72) Erfinder: AKRAM, Mustafa, D-22457 Hamburg (DE); KLEEN, Astrid, D-40699 Erkrath (DE); WOLFF, Wolfgang, D-22941 Bargteheide (DE)
(74) Vertreter: Foitzik, Joachim Kurt
(86) Internationale Anmeldenummer: EP9801062
(87) Internationale Veröffentlichungsnummer: WO98038976

(56) Entgegenhaltungen:
- DE-A- 3 247 806
- DE-A- 3 325 790
- FR-A- 2 290 186
- FR-A- 2 348 911
- FR-A- 2 514 258
- FR-A- 2 540 106

## Beschreibung

Die Erfindung betrifft Mittel zum Färben und Tönen keratinischer Fasern, insbesondere menschlicher Haare, mit speziellen direktziehenden Farbstoffen.

Zubereitungen zum Tönen und Färben von Haaren sind ein wichtiger Typ von kosmetischen Mitteln, Sie können dazu dienen, die natürliche Haarfarbe gemäß den Wünschen der entsprechenden Person leicht oder stärker zu nuancieren, eine gänzlich andere Haarfarbe zu erzielen oder unerwünschte Farbtöne, wie beispielsweise Grautöne, zu überdecken. Übliche Haarfärbemittel werden, je nach gewünschter Farbe bzw. Dauerhaftigkeit der Färbung, entweder auf Basis von Oxidationsfarbstoffen oder auf Basis von direktziehenden Farbstoffen formuliert. Häufig werden auch Kombinationen von Oxidationsfarbstoffen und direktziehenden Farbstoffen zur Erzielung spezieller Nuancen eingesetzt.

Färbemittel auf Basis von Oxidationsfarbstoffen führen zu brillanten und dauerhaften Farbtönen. Sie bedingen allerdings den Einsatz starker Oxidationsmittel wie beispielsweise Wasserstoffperoxid-Lösungen. Dies kann das zu färbende Haar schädigen. Diesen Schädigungen muß dann mit entsprechenden Pflegeprodukten entgegengewirkt werden. Außerdem können Kontakte der Haut mit diesen Färbemitteln bei sehr empfindlichen Personen zu unerwünschten Reaktionen führen.

Färbemittel auf Basis direktziehender Farbstoffe kommen ohne Oxidationsmittel aus und können besser bei pH-Werten im Bereich des Neutralpunktes formuliert werden. Ein wesentlicher Nachteil der Färbemittel auf Basis direktziehender Farbstoffe ist aber die geringe Waschechtheit der gefärbten Haare. Entsprechend können insbesondere das Aufziehvermögen der Farbstoffmoleküle auf das Haar sowie weiterhin der Glanz der gefärbten Haare in vielen Fällen nicht voll befriedigen.

Es besteht daher weiterhin das Bedürfnis nach Färbemitteln auf Basis direktziehender Farbstoffe, die sich durch ein verbessertes Aufziehvermögen der Farbstoffmoleküle auf das Haar und/oder einen verbesserten Glanz des gefärbten Haares auszeichnen.

Die FR-A-2 514 258 und die DE-A-3 247 806 offenbaren Haarfärbemittel, welche 1-Methoxy-3-nitro-4-(2-hydroxyethyl)amino-benzol enthalten.

Es wurde nun überraschenderweise gefunden, daß spezielle, dreifach substituierte Benzole die Anforderungen an direktziehende Farbstoffe in hervorragender Weise erfüllen. Die Verbindungen lassen sich sehr gut in übliche Färbemittelformulierungen einarbeiten und zeigen in einem breiten pH-Wert-Bereich eine sehr hohe Stabilität gegenüber Oxidationsmitteln. Die mit diesen Farbstoffen erzielten Ausfärbungen sind von einer außerordentlichen Brillanz bei gleichzeitig hoher Egalisierung und ausgezeichneter Licht-, Schweiß- und Waschechtheit. In diesem Zusammenhang sind insbesondere die Nuancen im Orangebereich zu nennen.

Gegenstand der Erfindung sind somit Mittel zum Färben oder Tönen keratinischer Fasern. insbesondere menschlicher Haare, dadurch gekennzeichnet, daß sie als direktziehenden Farbstoff eine Verbindung der Formel (I), 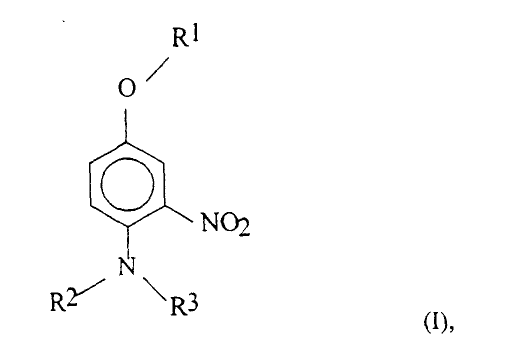 in der R¹ steht für eine C₁₋₄-Alkyl- oder eine C₅₋₇-Cycloalkylgruppe und R² und R³ unabhängig voneinander stehen für Wasserstoff, eine 3-Hydroxypropyl-, eine C₂₋₄-Di-, eine C₃₋₄-Trihydroxyalkylgruppe oder eine Amino-C₁₋₄-Alkylgruppe mit der Maßgabe, daß R² und R³ nicht gleichzeitig Wasserstoff sind, oder ein physiologisch verträgliches Salz dieser Verbindung enthalten.

Unter keratinischen Fasern sind erfindungsgemäß Pelze, Wolle. Federn und insbesondere menschliche Haare zu verstehen. Obwohl die erfindungsgemäßen Färbemittel in erster Linie zum Färben von keratinischen Fasern geeignet sind, steht prinzipiell einer Verwendung auch auf anderen Gebieten nichts entgegen.

Eine Reihe von Verbindungen der Formel (I) sind aus der Literatur bekannt. So wird beispielsweise in der DE-A1-33 25 790 das 1-Methoxy-3-nitro-4(3-hydroxypropylamino)benzol als Zwischenstufe bei der Herstellung der in dieser Druckschrift offenbarten Phenol-Farbstoffe erwähnt. Der Druckschrift ist aber nicht der geringste Hinweis auf irgendwelche Färbeeigenschaften dieser Zwischenprodukte zu entnehmen. Die DE-A1-32 47 806 offenbart eine Vielzahl von N-substituierten Nitroanilinen, die als direktziehende Farbstoffe in gängigen Oxidationsfärbemitteln dann verwendbar sind, wenn das alkalisch-reduzierende Milieu nicht ammoniakalisch ist und ein alkalisches Bisulfit sowie bestimmte Alkanolamine umfaßt. Informationen über die besonders vorteilhaften Eigenschaften der direktziehende Farbstoffe gemäß Formel (1) sind dieser Druckschrift nicht zu entnehmen.

In der Formel (I) steht R¹ für eine C₁₋₄-Alkylgruppe, beispielsweise für eine der Gruppen Methyl, Ethyl, Propyl, Isopropyl und Butyl. Ethyl und insbesondere Methyl sind bevorzugte Alkylgruppen. Die Cyclohexylgruppe ist eine bevorzugte C₅₋₇-Cycloalkylgruppe im Rahmen der Definition für R¹.

Erfindungsgemäß bevorzugt sind Verbindungen der Formel (I), bei denen R¹ für eine Alkylgruppe, insbesondere eine Methylgruppe, steht.

R² und R³ können unabhängig voneinander stehen für
- Wasserstoff,
- eine C₁₋₄-Alkylgruppe, z.B. eine Methyl-, Ethyl, n-Propyl-, iso-Propyl-, n-Butyl oder iso-Butylgruppe,
- eine 3-Hydroxypropylgruppe,
- eine C₂₋₄-Dihydroxyalkylgruppe, z.B. eine 2,3-Dihydroxypropylgruppe,
- eine C₃₋₄-Trihydroxyalkylgruppe, z.B. eine 2,3,4-Trihydroxybutylgruppe,
- eine Amino-C₁₋₄-Alkylgruppe. z.B. eine 2-Aminoethylgruppe.

Vom Schutzbegehren ausdrücklich ausgenommen sind solche Verbindungen der Formel (1), bei denen R² und R³ gleichzeitig für Wasserstoff stehen.

Erfindungsgemäß bevorzugt sind allerdings solche Verbindungen der Formel (I), bei denen R² für Wasserstoff steht.

Gleichfalls erfindungsgemäß bevorzugt sind solche Verbindungen der Formel (I), in denen R³ für eine 3-hydroxypropylgruppe eine C₂₋₄-Di- oder eine C₃₋₄-Trihydroxyalkylgruppe steht. Eine ganz besonders bevorzugte Gruppe R³ ist die 3-Hydroxypropylgruppe.

Eine Verbindung gemäß Formel (I), die sich hinsichtlich ihrer Eigenschaften als besonders hervorragend erwiesen hat, ist das 1-Methoxy-3-nitro-4(3-hydroxypropyl)aminobenzol.

Die Verbindungen der Formel (I) können den erfindungsgemäßen Mitteln als freie Basen zugegeben werden. Es ist aber auch möglich, diese Verbindungen in Form von physiologisch verträglichen Salzen mit anorganischen oder organischen Säuren zuzugeben. Solche Salze sind beispielsweise die Hydrochloride und die Sulfate. Der Begriff "Verbindungen gemäß Formel (I)" umfaßt daher stets sowohl die freien Basen als auch diese physiologisch verträglichen Salze.

Die erfindungsgemäßen Mittel enthalten die Verbindungen der Formel (1) üblicherweise in Mengen von 0,05 bis 5,0 Gew.-%, bezogen auf das Färbemittel, bei Oxidationsfärbemitteln ohne die Oxidationsmittelzubereitung. Mengen von 0,1 bis 3 Gew.-% sind bevorzugt.

Die Verwendbarkeit der Verbindungen gemäß Formel (I) in Mitteln zum Färben und Tönen von keratinischen Fasern unterliegt prinzipiell keinen Beschränkungen.

Gemäß einer ersten bevorzugten Ausführungsform der Erfindung handelt es um solche Mittel, die nur eine vorübergehende Färbung der Faser bewirken sollen. Solche Mittel werden häufig als Tönungsmittel bezeichnet, Diese Ausführungsform umfaßt beispielsweise auch solche Haarbehandlungsmittel, mit denen die Haare nicht nur vorübergehend gefärbt, sondern auch zu einer bestimmten Frisur gestylt werden sollen. In diesem Falle spricht man häufig von Tönungsfestigern.

Da solche Mittel üblicherweise ohne die Zuhilfenahme von oxidierenden Komponenten, insbesondere Wasserstoffperoxid, formuliert werden, sind die erfindungsgemäßen Mittel gemäß dieser Ausführungsform frei von Oxidationsfarbstoffvorprodukten. Wenngleich die Verbindungen gemäß Formel (I) auch als alleinige Farbstoffkomponente eingesetzt werden können, so enthalten die Mittel gemäß dieser Ausführungsform bevorzugt noch mindestens einen weiteren Farbstoff vom Typ der Direktzieher.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1. Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitrotoluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol.

Unter direktziehenden Farbstoffen werden erfindungsgemäß auch Naturfarbstoffe, wie beispielsweise Henna rot, Henna schwarz, Henna neutral, Kamillenblüte, Sandelholz, schwarzer Tee, Faulbaumrinde, Salbei, Blauholz, Krappwurzel, Catechu, Sedre und Alkannawurzel verstanden.

Die weiteren direktziehenden Farbstoffe sind in den erfindungsgemäßen Mitteln bevorzugt in Mengen von jeweils 0,01 bis 20 Gew.-%, bezogen auf das gesamte Mittel, enthalten.

Gemäß einer zweiten bevorzugten Ausführungsform handelt es sich bei den beanspruchten Mitteln um Haarfärbemittel für die dauerhafte Färbung der Haare. Diese Mittel enthalten mindestens ein Oxidationsfarbstoffvorprodukt vom Typ der Entwickler. Diese, in der Regel farblosen, Verbindungen reagieren unter der Einwirkung von Oxidationsmitteln oder von Luftsauerstoff, gegebenenfalls mit Hilfe spezieller Enzyme, mit sich selbst unter Ausbildung der gewünschten Farbstoffe. Insbesondere zur Ausbildung natürlicher Haarfarbtöne werden aber in der Regel Kombinationen von mehreren Entwicklerkomponenten eingesetzt. Weiterhin werden in der Regel zusätzlich sogenannte Kuppler eingesetzt, die unter dem Einfluß von Oxidationsmitteln mit den Entwicklerkomponenten reagieren, was zu neuen Farben bzw. einer Nuancierung der Farbe führt. Erfindungsgemäß kann sowohl eine Kupplerkomponente als auch mehrere Kupplerkomponenten in Kombination mit einer oder mehreren Entwickler-Komponenten eingesetzt werden.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylaminomethyl-4-amino-phenol, 4,4'-Diaminodiphenylamin, 4-Amino-3-fluorphenol, 2-Aminomethyl-4-amino-phenol, 2-Hydroxymethyl-4-aminophenol, Bis-(2-hydroxy-5-aminophenyl)-methan, 1,4-Bis-(4-aminophenyl)-diazacycloheptan. 1,3-Bis(N(2-hydroxyethyl)-N(4-aminophenylamino))-2-propanol, 4-Amino-2-(2-hydroxyethoxy)-phenol sowie 4,5-Diaminopyrazol-Derivate nach EP 0 740 741 bzw. WO 94/08970 wie z.B. 4,5-Diamino-1-(2'-hydroxyethyl)-pyrazol.

Ganz besonders bevorzugte Entwicklerkomponenten sind p-Phenylendiamin, p-Toluylendiamin, 2,4,5,6-Tetraaminopyrimidin, 4-Hydroxy-2,5,6-triaminopyrimidin, 2-Hydroxy-4,5,6-triaminopyrimidin, 1-(2'-Hydroxyethyl)-2,5-diaminobenzol und 4-Amino-3-methylphenol.

Als Kupplerkomponenten werden in der Regel m-Phenylendiaminderivate, Naphthole, Resorcin und Resorcinderivate, Pyrazolone und m-Aminophenole verwendet.

Erfindungsgemäß bevorzugte Kupplerkomponenten sind 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan. 4-Chlorresorcin, 2-Chlor-6-methyl-3-aminophenol, 2-Methylresorcin, 5-Methylresorcin, 2,5-Dimethylresorcin. 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin,
2,6-Dimethyl-3-amino-phenol, 3-Amino-6-methoxy-2-methylaminophenol, 2-Hydroxy-4-aminophenoxyethanol, 2-Methyl-5-(2-hydroxyethylamino)-phenol und 2,6-Dihydroxy-3,4-dimethylpyridin.

Ganz besonders bevorzugte Kupplerkomponenten sind m-Aminophenol, Resorcin, 2-Methylresorcin, 5-Methylresorcin, 2-Chlorresorcin, 4-Chlorresorcin und 2-Chlor-6-methyl-3-aminophenol.

Erfindungsgemäß können solche Entwickler- und Kupplerkomponemen, die für die Ausbildung der Färbungen keine Oxidationsmittel außer Luftsauerstoff benötigen, bevorzugt sein.

Die Entwickler- und Kupplerkomponenten werden üblicherweise in freier Form eingesetzt. Bei Substanzen mit Aminogruppen kann es aber bevorzugt sein, sie in Salzform. insbesondere in Form der Hydrochloride und Sulfate, einzusetzen.

Es ist nicht erforderlich. daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die erfindungsgemäßen Haarfarbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 2 enthalten sein können.

Die erfindungsgemäßen Mittel dieser Ausführungsform können außer den Verbindungen gemäß Formel (I), den Entwickler- und gegebenenfalls Kupplerkomponenten gewünschtenfalls zur Nuancierung noch weitere direktziehende Farbstoffe enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 6-Hydroxyindolin, 6-Aminoindolin und 4-Aminoindolin.

Bezüglich der in den erfindungsgemäßen Haarfärbe- und -tönungsmitteln einsetzbaren Farbstoffe wird weiterhin ausdrücklich auf die Monographie Ch. Zviak. The Science of Hair Care, Kapitel 7 (Seiten 248-250; direktziehende Farbstoffe) sowie Kapitel 8. Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.
Gemäß einer weiteren bevorzugten Ausführungsform enthalten die erfindungsgemäßen Mittel, insbesondere, wenn sie noch konditionierende oder festigende Eigenschaften aufweisen sollten, noch anionische, nichtionogene oder insbesondere kationische Polymere.

Als konditionierende Wirkstoffe sind beispielsweise die im folgenden beschriebenen kationischen Polymeren geeignet:

Die kationischen Polymeren enthalten innerhalb des Polymergerüstes kationische Gruppen. Diese Gruppen können Teil der Polymerkette sein, sie können sich aber auch in Seitenketten befinden, die über Zwischenglieder mit einer Hauptkette verbunden sind. Übliche kationische Gruppen enthalten quartäre Stickstoff- oder Phosphoratome. Gruppen mit quartären Stickstoffatomen sind dabei bevorzugt. Die quartären Stickstoffatome können dabei sowohl 4 unterschiedliche oder z.T. gleiche Substituenten tragen, als auch Teil eines Ringsystems sein. Bevorzugte kationische Gruppen sind Ammonium- und Imidazoliniumgruppen.

Beispiele für solche Polymere sind:
- quatemierte Cellulose-Derivate, wie sie unter den Bezeichnungen Celquat^{R} und Polymer JR^{R} im Handel erhältlich sind. Die Verbindungen Celquat H 100, Celquat L 200 und Polymer JR^{R}400 sind bevorzugte quaternierte Cellulose-Derivale.
- quaternierte Guar-Derivate, wie sie unter den Bezeichnungen Cosmedia Guar^{R} und Jaguar^{R} im Handel erhältlich sind. Bevorzugte Guar-Derivate sind beispielsweise Cosmedia Guar^{R} C-261 und Jaguar^{R} C 13-S.
- Copolymere des Vinylpyrrolidons mit quaternierten Derivaten des Dialkylaminoalkylacrylats- und -methacrylats, wie beispielsweise mit Diethylsulfat quaternierte Vinylpyrrolidon-Dimethylaminoalkylmethacrylat-Copolymere sowie das Vinylpyrrolidon-Methacrylamidopropyltrimethylamrnoniumchlorid-Copolymere. Solche Verbindungen sind unter den Bezeichnungen Gafquat^{R}734, Gafquat^{R}755 bzw. Gafquat^{R} HS100 im Handel erhältlich.
- Copolymerisate des Vinylpyrrolidons mit Vinylimidazoliummethochlorid, wie sie unter der Bezeichnung Luviquat^{R} angeboten werden.
- Polymere Dimethyldiallylammoniumsalze und deren Copolymere mit Estern und Amiden von Acrylsäure und Methacrylsäure. Die unter den Bezeichnungen Merquat^{R}100 (Poly(dimethyldiallylammoniumchlorid)) und Merquat^{R}550 (Dimethyldiallylammoniumchlorid-Acrylamid-Copolymer) im Handel erhältlichen Produkte sind Beispiele für solche kationischen Polymere.
- Kationisch derivatisierte Silikonöle, wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{R}-Quat 3270 und 3272 (Hersteller: Th. Goldschmidt; diquaternäre Polydimethylsiloxane, Quaternium-80).
- Chitosan und dessen Derivate

Bevorzugt sind die kationischen Polymeren in den erfindungsgemäßen Mitteln in Mengen von 0,1 - 5 Gew.-%. bezogen auf die gesamte Zubereitung, enthalten.

Geeignete nichtionogene Polymere sind beispielsweise:
- Vinylpyrrolidon/Vinylester-Copolymere, wie sie beispielsweise unter dem Warenzeichen Luviskol^{R} (BASF) vertrieben werden. Luviskol^{R} VA 64 und Luviskol^{R} VA 73, jeweils Vinylpyrrolidon/Vinylacetat-Copolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{R} und Benecel^{R} (AQUALON) vertrieben werden.
- Schellack
- Polyvinylpyrrolidone, wie sie beispielsweise unter der Bezeichnung Luviskol^{R} (BASF) vertrieben werden.

Beispiele für geeignete anionische Polymere sind:
- Copolymere der Acrylsäure und/oder Methacrylsäure oder deren Ester mit C₁₀₋₃₀-Alkylacrylaten, wie sie beispielsweise unter der Bezeichnung Pemulen^{R} vertrieben werden.
- Polymere und Copolymere der Crotonsäure mit Estern und Amiden der Acryl- und der Methacrylsäure, wie Vinylacetat-Crotonsäure- und Vinylacetat-Vinylpropionat-Crotonsäure-Copolymere. Verbindungen dieser Art sind unter den Markenbezeichnungen Resyn^{R} (NATIONAL STARCH), Luviset^{R} (BASF) und Gafset^{R} (GAF) im Handel; die Produkte Luviset^{R}CA-66 und Luviset^{R}CAP können bevorzugt sein.
- Vinylpyrrolidon/Vinylacrylat-Copolymere, erhältlich beispielsweise unter dem Warenzeichen Luviflex^{R} (BASF). Ein bevorzugtes Polymer ist das unter der Bezeichnung Luviflex^{R} VBM-35 (BASF) erhältliche Vinylpyrrolidon/Acrylat-Terpolymere.
- Acrylsäure/Ethylacrylat/N-tert.Butylacrylamid-Terpolymere, die beispielsweise unter der Bezeichnung Ultrahold^{R} strong (BASF) vertrieben werden, sowie Methacrylsäure/Ethylacrylat/t-Butylacrylat-Terpolymere, die unter der Bezeichnung Luvimer^{R}100P (BASF) vertrieben werden.

Zur Herstellung der erfindungsgemäßen Färbemittel werden die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen. Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist.
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet. die im Molekül mindestens eine quartäre Ammoniumgruppe und mindestens eine-COO⁽⁻⁾ - oder -SO ₃⁽⁻⁾-Gruppe tragen. Besonders geeignete zwitterionische Tenside sind die sogenannten Betaine wie die N-Alkyl-N.N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl.
- Anlagerungeprodukte von Ethylenoxid an Sorbitanfettsäureester und
- Anlagerungsprodukte von Ethylenoxid an Fettsäurealkanolamide.

Beispiele für die in den erfindungsgemäßen Mitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Dialkylammoniummethosulfate und Methyl-hydroxyalkyldialkoyloxyalkyl-ammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen. Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide,
-hydroxide oder -alkoholate als Katalysatoren verwendet werden. Die Verwendung von Produkten mit eingeengter Homologenverteilung kann bevorzugt sein.

Die Tenside liegen in den erfindungsgemäßen Mitteln bevorzugt in Mengen von 1 - 20, insbesondere etwa 6 - 10 Gew.-%, jeweils bezogen auf das gesamte Mittel, vor, wenn es sich bei dem erfindungsgemäßen Mittel um ein Färbeshampoo handelt. Bei Färbelotionen oder Färbegelen sind dagegen Mengen von 1 - 10, insbesondere 2 - 4 Gew.-%, jeweils bezogen auf das gesamte Mittel, bevorzugt.

Weitere Wirk-, Hilfs- und Zusatzstoffe sind beispielsweise
- zwitterionische und amphotere Polymere wie beispielsweise Acrylamidopropyltrimethylammoniumchlorid/Acrylat-Copolymere und Octylacrylamid/Methylmethacrylat/tert.Butylaminoethylmethacrylat/2-Hydroxypropylmethacrylat-Copolymere,
- Verdickungsmittel wie Agar-Agar, Guar-Gum, Alginate, Pektine, Xanthan-Gum, Gummi arabicum. Karaya-Gummi, Johannisbrotkernmehl. Leinsamengummen, Dextrane. Cellulose-Derivate, z. B. Methylcellulose, Hydroxyalkylcellulose und Carboxymethylcellulose, Stärke-Fraktionen und Derivate wie Amylose. Amylopektin und Dextrine, Tone wie z. B. Bentonit oder vollsynthetische Hydrokolloide wie z.B. Polyvinylalkohol.
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren, quaternisierte Proteinhydrolysate und Aminosäuren,
- Parfümöle, Dimethylisosorbid und Cyclodextrine,
- Lösungsvermittler wie Ethanol, Isopropanol, Ethylenglykol, Propylenglykol, Glycerin, Diethylenglykol und Methoxybutanol,
- Farbstoffe zum Einfärben der Zubereitungen,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze. Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat. Bienenwachs, Montanwachs, Paraffine, Fettalkohole und Fettsäureester,
- Fettsäurealkanolamide.
- Komplexbildner wie Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO und Luft
- Reduktionsmittel wie z.B. Thioglykolsäure und deren Derivate, Thiomilchsäure, Cysteamin, Thioäpfelsäure, α-Mercaptoethansulfonsäure, Natriumsulfit, Ascorbinsäure und Natriumdithionit,
- Oxidationsmittel wie Wasserstoffperoxid, Kaliumbromat und Natriumbromat.
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0.5 bis 30 Gew.-% und Verdickungsmittel in Konzentrationen von 0,1 bis 25 Gew.-% des gesamten Färbemittels eingesetzt.

Gegenstand der Anmeldung ist auch die Verwendung eines erfindungsgemäßen Mittels zum Färben und Tönen von keratinischen Fasern, insbesondere menschlichen Haaren.

Es ist prinzipiell möglich, die erfindungsgemäßen Mittel so zu formulieren, daß sie entweder auf dem Haar verbleiben können oder wieder aus dem Haar ausgespült werden.

Gemäß einer bevorzugten Ausführungsform werden die erfindungsgemäßen Mittel so formuliert, daß sie auf dem Haar verbleiben können. Dies ist insbesondere bei sogenannten Tönungsmitteln der Fall, aber auch bei Mitteln, die außerdem eine festigende Funktion ausüben sollen.

Die nachfolgenden Beispiele sollen den Erfindungsgegenstand näher erläutern.

### Ausführungsheispiele

Alle Mengenangaben in den Beispielen sind Gewichtsteile.
1. Tönungsmittel

| | |
|---|---|
| Monoethanolaminlaurylsulfat | 5,00 |
| Cetylalkohol | 1,00 |
| Diethylenglykolmonoethylether | 5,00 |
| 1-Methoxy-3-nitro-4(3-hydroxypropyl)aminobenzol | 0,65 |
| Hydroxyethylcellulose | 0,90 |
| Wasser | ad 100,00 |

Dieses Mittel wurde auf hellblondes Human-Haar aufgebracht, dort 30 Minuten bei Raumtemperatur belassen und danach mit klarem Wasser gut ausgespült. Nach dem Trocknen hatte das so behandelte Haar einen rotorangen Ton erhalten.
2. Haarfärbecreme

| | |
|---|---|
| Lauryldiglykolethersulfat, Natriumsalz | 4,20 |
| Cetylalkohol | 16,00 |
| 1-Methoxy-3-nitro-4(3-hydroxypropyl)aminobenzol | 0,30 |
| p-Toluylendiaminsulfat | 0,20 |
| Resorcin | 0,10 |
| m-Aminophenol | 0,01 |
| 4-Chlorresorcin | 0,07 |
| Ammoniak (25 %ig) | 7,50 |
| Natriumsulfit | 0,20 |
| Wasser | ad 100,00 |

Das Mittel wurde im Gewichtsverhältnis 1:1 mit einer 6 Gew.-% wäßrigen Wasserstoffperoxidlösung vermischt und auf graues Human-Haar aufgetragen. Nach einer Einwirkzeit von 30 Minuten bei Raumtemperatur wurde das Haar gut ausgespült, nachshampooniert und getrocknet. Das Haar hatte nun einen hellgoldblonden Ton.
3. Haarfestiger

| | |
|---|---|
| Polyvinylpyrrolidon | 2,50 |
| Glycerin | 0,12 |
| 1-Methoxy-3-nitro-4(3-hydroxypropyl)aminobenzol | 0,08 |
| Isopropanol | 38,00 |
| Wasser | ad 100,00 |

Mittelblondes, nicht ergrautes Natur-Haar wurde nach dem Aufbringen des Festigers eingelegt und getrocknet. Das Haar war dadurch goldblond eingefärbt und gefestigt.
4. Cremecoloration

| | |
|---|---|
| Eumulgin® B3¹ | 2,30 |
| Cetylalkohol | 4,50 |
| Stearylalkohol | 4,00 |
| Cutina®GMS² | 0,50 |
| Eumulgin® B2³ | 1.00 |
| 1-Methoxy-3-nitro-4(3-hydroxypropyl)aminobenzol | 0.30 |
| p-Toluylendiaminsulfat | 0.50 |
| Resorcin | 0,12 |
| 2-Methylresorcin | 0,04 |
| m-Aminophenol | 0,03 |
| 4-Chlorresorcin | 0,06 |
| Natriumsulfit | 0,30 |
| Monoethanolamin | ad pH 8.5 |
| Wasser | ad 100,00 |

| | |
|---|---|
| ¹ Cetylstearylalkohol mit ca. 30 Mol Ethylenoxid (INCI-Bezeichnung: Ceteareth-30) (HENKEL) | |
| ² Glycerinmonostearat (INCI-Bezeichnung: Glyceryl Stearate) (HENKEL) | |
| ³ Cetylstearylalkohol mit ca. 20 Mol Ethylenoxid (INCI-Bezeichnung: Ceteareth-20) (HENKEL) | |

Die Cremecoloration wurde im Gewichts-Verhältnis 1:2 mit einer 1,5 %igen wäßrigen Wasserstoffperoxidlösung angerührt, auf graues Human-Haar aufgetragen und dort 30 Minuten bei Raumtemperatur belassen. Das Haar wurde dann gut gespült, nachshampooniert und getrocknet. Es hatte dann eine hellgoldblonde Färbung.

## Patentansprüche

1. Mittel zum Färben oder Tönen keratinischer Fasern, insbesondere menschlicher Haare, **dadurch gekennzeichnet, daß** es als direktziehenden Farbstoff eine Verbindung der Formel (I), 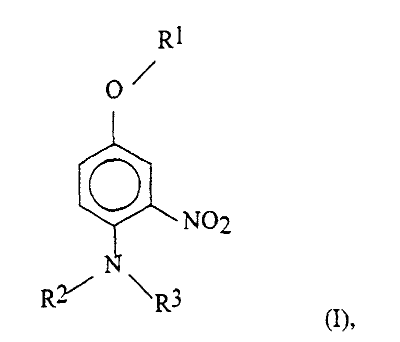 in der R¹ steht für eine C₁₋₄-Alkyl- oder eine C₅₋₇-Cycloalkylgruppe, und R² und R³ unabhängig voneinander stehen für Wasserstoff, eine 3-Hydroxypropyl-, eine C₂₋₄-Di- oder eine C₃₋₄-Trihydroxyalkylgruppe mit der Maßgabe, daß R² und R³ nicht gleichzeitig Wasserstoff sind, oder ein physiologisch verträgliches Salz dieser Verbindung enthält.

2. Mittel nach Anspruch 1, **dadurch gekennzeichnet, daß** in der Verbindung der Formel (I) R¹ steht für eine C₁₋₄-Alkylgruppe, R² für Wasserstoff und R³ für eine 3-Hydroxypropyl-, eine C₂₋₄-Di- oder eine C₃₋₄-Trihydroxyalkylgruppe.

3. Mittel nach Anspruch 1 oder 2, **dadurch gekennzeichnet, daß** R¹ eine Methylgruppe und R³ eine 3-Hydroxypropylgruppe darstellt.

4. Mittel nach einem der Ansprüche 1 bis 3, **dadurch gekennzeichnet, daß** es frei von Oxidationsfarbstoffvorprodukten ist.

5. Mittel nach einem der Ansprüche 1 bis 4, **dadurch gekennzeichnet, daß** es weiterhin ein kationisches Polymer enthält.

6. Mittel nach einem der Ansprüche 4 und 5, **dadurch gekennzeichnet. daß** es auf dem Haar verbleibt.

7. Mittel nach einem der Ansprüche 4 bis 6, **dadurch gekennzeichnet, daß** es sich um ein haartönendes oder haarfestigendes Mittel handelt.

8. Mittel nach einem der Ansprüche 1 bis 3, **dadurch gekennzeichnet, daß** es weiterhin mindestens eine Entwicklerkomponente und / oder mindestens eine Kuppler-Komponente enthält.

9. Mittel nach Anspruch 8, **dadurch gekennzeichnet, daß** die Entwicklerkomponente ausgewählt ist aus p-Phenylendiamin, p-Toluylendiamin, 2,4,5,6-Tetraaminopyrimidin, 4-Hydroxy-2,5,6-triaminopyrimidin, 2-Hydroxy-4,5,6-triaminopyrimidin, 1-(2'-Hydroxyethyl)-2.5-diaminobenzol und 4-Amino-3-methylphenol.

10. Mittel nach Anspruch 8 oder 9, **dadurch gekennzeichnet, daß** die Kupplerkomponente ausgewählt ist aus m-Aminophenol, Resorcin, 2-Methylresorcin, 5-Methylresorcin, 2-Chlorresorcin, 4-Chlorresorcin und 2-Chlor-6-methyl-3-aminophenol.

11. Verwendung eines Mittels nach einem der Ansprüche 1 bis 10 zum Färben oder Tönen von Haaren.

## Claims

1. A composition for colouring or tinting keratin fibers, more especially human hair, **characterized in that** it contains a compound corresponding to formula (I): 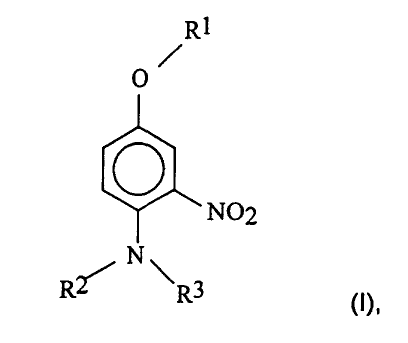 in which R¹ is a C₁₋₄ alkyl group or a C₅₋₇ cycloalkyl group and R² and R³ independently of one another represent hydrogen, a 3-hydroxypropyl group, a C₂₋₄ dihydroxyalkyl group or a C₃₋₄ trihydroxyalkyl group, with the proviso that R² and R³ are not both hydrogen, or a physiologically compatible salt of this compound as a substantive dye.

2. A composition as claimed in claim 1, **characterized in that**, in the compound of formula (I), R¹ is a C₁₋₄ alkyl group, R² is hydrogen and R³ is a 3-hydroxypropyl group, a C₂₋₄ dihydroxyalkyl group or a C₃₋₄ trihydroxyalkyl group.

3. A composition as claimed in claim 1 or 2, **characterized in that** R¹ is a methyl group and R³ is a 3-hydroxypropyl group.

4. A composition as claimed in any of claims 1 to 3, **characterized in that** it is free from oxidation dye precursors.

5. A composition as claimed in any of claims 1 to 4, **characterized in that** it additionally contains a cationic polymer.

6. A composition as claimed in claim 4 or 5, **characterized in that** it remains on the hair.

7. A composition as claimed in any of claims 4 to 6, **characterized in that** it is formulated as a hair tint or a hair setting composition.

8. A composition as claimed in any of claims 1 to 3, **characterized in that** it additionally contains at least one primary intermediate and/or at least one secondary intermediate.

9. A composition as claimed in claim 8, **characterized in that** the primary intermediate is selected from p-phenylenediamine, p-toluylenediamine, 2,4,5,6-tetraminopyrimidine, 4-hydroxy-2,5,6-triaminopyrimidine, 2-hydroxy-4,5,6-triaminopyrimidine, 1-(2'-hydroxyethyl)-2,5-diaminobenzene and 4-amino-3-methylphenol.

10. A composition as claimed in claim 8 or 9, **characterized in that** the secondary intermediate is selected from m-aminophenol, resorcinol, 2-methyl resorcinol, 5-methyl resorcinol, 2-chlororesorcinol, 4-chlororesorcinol and 2-chloro-6-methyl-3-aminophenol.

11. The use of the compositions claimed in any of claims 1 to 10 for colouring or tinting hair.

## Revendications

1. Agent pour teindre ou nuancer les fibres kératiniques, en particulier les cheveux humains,
**caractérisé en ce qu'**
il contient comme colorant à action directe un composé de formule (I) : 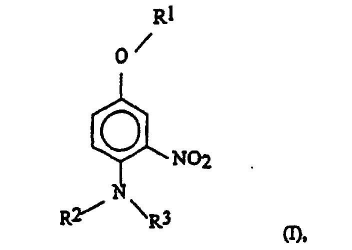 dans laquelle R¹ représente un groupe alkyle en C₁-C₄ ou un groupe cycloalkyle en C₅-C₇ et R² et R³ indépendamment l'un de l'autre, représentent de l'hydrogène, un groupe 3-hydroxypropyle, un groupe allyle dihydroxylé en C₂-C₄ ou un groupe alkyle trihydroxylé en C₃-C₄ avec la précision que R² et R³ ne sont pas simultanément de l'hydrogène, ou un sel physiologiquement compatible de ce composé.

2. Agent selon la revendication 1,
**caractérisé en ce que**
dans le composé de formule (I), R¹ représente un groupe alkyle en C₁-C₄, R² représente de l'hydrogène, et R³ représente un groupe 3-hydroxypropyle, un groupe alkyl dihydroxylé en C₂-C₄ ou un groupe alkyle trihydroxylé en C₃-C₄.

3. Agent selon la revendication 1 ou la revendication 2,
**caractérisé en ce que**
R¹ représente un groupe méthyle et R³ représente un groupe 3-hydroxypropyle.

4. Agent selon l'une quelconque des revendications 1 à 3,
**caractérisé en ce qu'**
il est dépourvu de produits précurseurs de colorant par oxydation.

5. Agent selon l'une quelconque des revendications 1 à 4,
**caractérisé en ce qu'**
il contient en outre un polymère cationique.

6. Agent selon l'une quelconque des revendications 4 et 5,
**caractérisé en ce qu'**
il demeure sur les cheveux.

7. Agent selon l'une quelconque des revendications 4 à 6,
**caractérisé en ce qu'**
il s'agit d'un agent qui nuance les cheveux ou d'un agent qui solidifie les cheveux.

8. Agent selon l'une quelconque des revendications 1 à 3,
**caractérisé en ce qu'**
il contient en outre au moins un composant d'agent révélateur et/ou au moins un composant d'agent de couplage.

9. Agent selon la revendication 8,
**caractérisé en ce que**
le composant d'agent révélateur est choisi parmi la p-phénylènediamine, la p-totuylènediamine, la 2,4,5,6-tétraaminopyrimidine, la 4-hydroxy-2,5,6-triaminopyrimidine, la 2-hydroxy-4,5,6-triaminopyrimidine, le 1-(2',hydroxyéthyl)2,5-diaminobenzène, et le 4-amino-3-méthylphénol.

10. Agent selon la revendication 8 ou la revendication 9,
**caractérisé en ce que**
le composant d'agent de couplage est choisi parmi le m-aminophénol, le résorcinol, le 2-méthylrésorcinol, le 5-méthylrésorcinol, le 2-chlororésorcinol, le 4-chlororésorcinol et le 2-chloro-6-méthyl-3-aminophénol.

11. Utilisation d'un agent selon l'une quelconque des revendications 1 à 10, pour teindre ou colorer des cheveux.
